# DEMANDE DE BREVET EUROPEEN

(11) **EP 1 270 536 A2**
(43) Date de publication de la demande: **02.01.2003**
(21) Numéro de dépôt: 02078805.5
(22) Date de dépôt: 21.01.1997
(51) Int. Cl.: C07C 19/01, C07C 17/278, C07C 19/08, C07C 17/20

(54) **Preparation du 1,1,1,3,3-pentachlorobutane et du 1,1,1,3,3-pentafluorobutane**

(30) Priorité: 01.02.1996 FR 9601214
(62) Demande divisionnaire de: 97400119.0
(71) Demandeur: Atofina, 92800 Puteaux (FR)
(72) Inventeur: Bertocchio, René, 69390 Vourles (FR); Lantz, André, 69390 Vernaison (FR); Wendlinger, Laurent, 69530 Brignais (FR)
(74) Mandataire: Dang, Doris

(57) **Abrégé**

Le 1,1,1,3,3-pentachlorobutane dont la fluoration conduit au 1,1,1,3,3-pentafluorobutane, est préparé avec un rendement et une sélectivité élevés par addition du tétrachlorure de carbone sur le 2-chloropropène en présence d'un sel de cuivre et d'une amine.

## Description

La présente invention concerne le domaine des hydrocarbures halogénés et a plus particulièrement pour objet la préparation du 1,1,1,3,3-pentachlorobutane et sa fluoration en 1,1,1,3,3-pentafluorobutane.

Avec un point d'ébullition de 40°C, le 1,1,1,3,3-pentafluorobutane (connu sous la désignation F365 mfc) est un substitut potentiel des chlorofluoroalcanes liquides bannis par le protocole de Montréal, tout particulièrement le fluorotrichloro-méthane (F11 ; Eb. = 27°C) et le trichlorotrifluoroéthane (F113 ; Eb. = 47°C).

Les procédés permettant de préparer le F365 mfc sont peu nombreux et font généralement appel à la fluoration d'un précurseur chloré tel que le 2,2-difluoro-4,4,4-trichlorobutane, le 2-bromo-2,4,4,4-tétrachlorobutane et le 1,1,1,3,3-pentachlorobutane.

Henne et coll. (J. Am. Chem. Soc. 67, p. 1194-1197 et 1197-1199, 1945) chlorent le 2,2-difluorobutane pour obtenir avec une sélectivité de 52,5 % le 2,2-difluoro-4,4,4-trichlorobutane (F362 jfc) qui est ensuite fluoré en F365 mfc. McBee et Hausch (Ind. Eng. Chem. 39, p.418-420, 1947) fluorent le F362 jfc par HF/HgO ou par le mélange SbF₃/SbCl₅, mais les rendements de fluoration ne dépassent pas 15 %. Tous ces procédés se caractérisent par de faibles rendements dûs essentiellement au manque de sélectivité des réactions de chloration conduisant au précurseur chloré.

Une autre méthode de préparation du F365 mfc, décrite par Tarrant et Coll. (J. Am. Chem. Soc. 80, p.1711-1713, 1958) consiste en l'addition radicalaire de CCl₃Br sur le 2-chloropropène et la fluoration par HF, en l'absence de catalyseur, du produit d'addition 1:1 (CCl₃CH₂CBrClCH₃) obtenu avec un rendement de 34 %. Cette méthode n'améliore pas le rendement global puisque, dans ce cas, on observe un manque de sélectivité dû à la formation de télomères favorisée par l'initiation radicalaire au peroxyde de benzoyle.

Un autre précurseur chloré du F365 mfc est le 1,1,1,3,3-pentachlorobutane qui, selon Friedlina et Coll. [Izv. Akad. Nauk SSSR (6), p.1333-1336 (1980) et (8), p.1903-5 (1979)], peut être obtenu par télomérisation du chlorure de vinylidène par le 1,1,1-trichloroéthane ou celle du 2-chloropropène par CCl₄ en présence de fer pentacarbonyle. Dans les deux cas, il est obtenu un mélange de trois télomères et la sélectivité en 1,1,1,3,3-pentachlorobutane (produit d'addition 1:1) est insuffisante pour présenter un intérêt économique certain.

La préparation directe du F365 mfc selon la méthode de Bloshchitsa et Coll. (Zhur. Org. Khim 24(7), p. 1558, 1988) par action de l'acide fluorhydrique et du tétrafluorure de soufre sur le dicétène, constitue en fait la seule méthode conduisant au F365 mfc avec un rendement acceptable (70 %). Malheureusement, ce procédé fait appel à deux matières premières d'usage peu courant, le dicétène et le tétrafluorure de soufre.

Le F365 mfc est aussi un sous-produit de la fabrication du 1,1-dichloro-1-fluoroéthane (F141b), mais la proximité de leurs points d'ébullition (F141b : Eb. = 32°C ; F365 mfc : Eb. = 40°C) et l'existence d'un mélange azéotropique à point d'ébullition minimum ne permettent pas une séparation facile des deux produits. Un procédé de séparation, basé sur le principe d'une distillation en présence d'un excès d'HF, est cependant décrit dans le brevet EP 395 793.

Les complexes amine/sel cuivreux sont des catalyseurs connus pour l'addition de composés polyhalogénés sur les oléfines (Kotora et Coll., J. of Molecular Catalysis 77, p.51-61, 1992), mais ces auteurs montrent que le rendement en produit d'addition 1:1 peut passer de 97 % pour le chlorure de vinyle à 11 % pour le 1,2-dichloroéthylène.

En recherchant un procédé permettant de préparer le F365 mfc avec un rendement et une sélectivité élevés, il a maintenant été trouvé que le 2-chloropropène se classe parmi les oléfines réactives vis-à-vis du tétrachlorure de carbone et peut, dans certaines conditions, conduire au produit d'addition 1:1 attendu, le 1,1,1,3,3-pentachlorobutane, avec un excellent rendement et une excellente sélectivité, la formation de sous-produits, télomères ou produits d'élimination étant négligeable. De plus, parmi ces sous-produits, les oléfines C₄H₄Cl₄ éventuellement formées par déshydrochloration du 1,1,1,3,3-pentachlorobutane se fluorent facilement en 1,1,1,3,3-pentafluorobutane.

L'invention a donc pour premier objet un procédé de préparation du 1,1,1,3,3-pentachlorobutane par addition du tétrachlorure de carbone sur le 2-chloropropène, caractérisé en ce que l'on opère en présence d'un sel de cuivre et d'une amine.

L'invention a également pour objet un procédé de préparation du F365 mfc comprenant une première étape d'addition du tétrachlorure de carbone sur le 2-chloropropène et une étape de fluoruration par l'acide fluorhydrique du 1,1,1,3,3-pentachlorobutane ainsi obtenu.

Comme sel de cuivre, on utilise avantageusement un sel cuivreux, de préférence un halogénure et plus particulièrement le chlorure cuivreux.

L'amine à utiliser peut être une mono-, di- ou trialkylamine dont le radical alkyle ou les radicaux alkyle, linéaire(s) ou ramifié(s), peuvent contenir de 1 à 8 (de préférence 2 à 4) atomes de carbone. On peut également utiliser une amine cyclanique, par exemple la cyclohexylamine. On utilise avantageusement une amine primaire et, plus particulièrement, l'isopropylamine.

Le 2-chloropropène peut être obtenu de façon connue en soi, par déshydrochloration du 1,2-dichloropropane ou du 2,2-dichloropropane (thermiquement ou par action de la potasse glycolique) ou, de préférence, par action du phénylchloroforme sur l'acétone en présence d'un acide de Lewis comme le chlorure de zinc ou le chlorure ferrique (brevet FR 2 213 257) ; dans ce cas, le rendement en 2-chloropropène atteint 77 % et le principal sous-produit de la réaction, le 2,2-dichloropropane, peut aisément être converti en 2-chloropropène par élimination d'HCI selon le procédé à la potasse ou à la soude glycolique décrit dans le brevet US 2 543 648.

Le rapport molaire CCl₄/CH₃CCl = CH₂ peut aller de 2 à 6, mais il est de préférence compris entre 2,5 et 4,5.

Le sel de cuivre, en particulier le chlorure cuivreux, a pour rôle d'amorcer la formation du radical trichlorométhyle et d'assurer le transfert du chlore sur le radical CCl₃CH₂CClCH₃ résultant de l'addition du radical CCl₃ sur le 2-chloropropène. Il est généralement utilisé en une quantité telle que le rapport molaire : sel de cuivre/2-chloropropène soit compris entre 0,001 et 0,05, de préférence entre 0,005 et 0,02.

La concentration en amine influe de manière prépondérante sur le rendement en 1,1,1,3,3-pentachlorobutane. Rapportée au nombre de moles total du mélange réactionnel initial (CCl₄ + CH₃CCl = CH₂ + CuCI + amine), elle peut aller de 0,5 à 10 %, mais elle est de préférence comprise entre 1 et 8 % et, plus particulièrement, entre 2,5 et 6 %.

La réaction peut être effectuée à une température comprise entre 80 et 130°C, mais on opère de préférence entre 90 et 110°C.

Le 1,1,1,3,3-pentachlorobutane formé qui peut être séparé du mélange réactionnel par des procédés connus en soi, notamment par filtration, lavage acide, lavage à l'eau, séchage et distillation, est utilisé conformément au second aspect de la présente invention pour la synthèse du 1,1,1,3,3-pentafluorobutane par fluoruration au moyen d'acide fluorhydrique.

Cette opération peut être réalisée en phase liquide, en présence ou en l'absence de catalyseur. Elle est généralement effectuée sous pression autogène à une température comprise entre 80 et 120°C, de préférence d'environ 100°C. Le rapport molaire HF/CCl₃CH₂CCl₂CH₃ peut aller de 15 à 30, mais il est avantageusement compris entre 20 et 25. Comme catalyseur, on peut utiliser n'importe quel catalyseur de fluoruration en phase liquide, en particulier un catalyseur à base d'antimoine. Les meilleurs résultats ont cependant été obtenus sans catalyseur.

Les exemples suivants illustrent l'invention sans la limiter.

### EXEMPLE 1

Dans un tube en Pyrex à paroi épaisse maintenu à 0°C, on a introduit successivement 8 mg de chlorure cuivreux, 117 mg de n.butylamine, 370 mg de 2-chloropropène (préalablement déstabilisé par distillation) et enfin 3,2 g de tétrachlorure de carbone. Le tube a alors été scellé, homogénéisé et chauffé à température constante (100°C) durant 4 heures, sous agitation.

Après refroidissement, le tube a été ouvert sur une rampe à gaz permettant de récupérer quantitativement la phase gazeuse, la phase liquide étant transvasée dans un flacon pour une analyse séparée. Les deux phases ont été analysées par chromatographie en phase gazeuse et le bilan de la réaction reconstitué à partir de ces analyses.

Les résultats de cet essai (Essai 1-a), ainsi que les conditions opératoires et les résultats obtenus avec d'autres amines (Essais 1-b à 1-g), sont résumés dans le tableau (I) où les abréviations ont les significations suivantes :
T.T. : taux de transformation
360 jfa : 1,1,1,3,3-pentachlorobutane

**TABLEAU I**

| **ESSAI N°** | **1-a** | **1-b** | **1-c** | **1-d comparatif** | **1-e** | **1-f** | **1-g** |
|---|---|---|---|---|---|---|---|
| **Amine** | **n.butylamine** | **isopropylamine** | **tertbutylamine** | **éthanolamine** | **diéthylamine** | **triéthylamine** | **cyclohexylamine** |
| ***Conditions opératoires* :** | | | | | | | |
| . concentration en amine (% molaire) | 5,9 | 5 | 3,35 | 1,24 | 4 | 4,7 | 6,0 |
| . rapport molaire CCl₄/ 2-chloropropène | 4,3 | 4 | 4 | 4 | 4 | 4,1 | 3,9 |
| . rapport molaire CuCl/2-chloropropène | 0,017 | 0,014 | 0,014 | 0,01 | 0,02 | 0,02 | 0,016 |
| ***Résultats* :** | | | | | | | |
| T.T. (%) du 2-chloropropène | 99,7 | 98,4 | 99,3 | 2,7 | 84,3 | 57,1 | 95,4 |
| Sélectivité (%) en : | | | | | | | |
| - 360 jfa | 90.7 | 94.2 | 85.7 | 0.8 | 68 | 37.1 | 90.3 |
| - oléfines | 1.8 | 0.3 | 3.8 | 0.4 | 0.2 | 1 | 1.3 |
| - lourds | 7.2 | 3.8 | 9.9 | 1.6 | 16.1 | 19 | 9.4 |
| T.T. (%) du CCl₄ en CHCl₃ | 1,8 | 1 | 0,3 | 1 | 0,7 | 0,7 | 1,1 |

### EXEMPLE 2

On a opéré comme à l'exemple 1 avec l'isopropylamine, mais en faisant varier sa concentration dans le mélange réactionnel.

Les résultats sont rassemblés dans le tableau II suivant.

**TABLEAU II**

| **ESSAI N°** | **2-a** | **2-b** | **2-c(*)** | **1-b** | **2-d(**)** | **2-e** |
|---|---|---|---|---|---|---|
| ***Conditions opératoires* :** | | | | | | |
| . concentration en isopropylamine (% molaire) | 0,5 | 1,0 | 2,6 | 5,0 | 6,9 | 7,5 |
| . rapport molaire CCl₄/ 2-chloropropène | 4,4 | 4,2 | 4,1 | 4,0 | 4,1 | 3,9 |
| . rapport molaire CuCl/2-chloropropène | 0,0136 | 0,0094 | 0,0197 | 0,014 | 0,0310 | 0,0130 |
| ***Résultats* :** | | | | | | |
| T.T. du 2-chloropropène (%) | 1 | 54 | 97,8 | 98,4 | 79,3 | 57,4 |
| Sélectivité (%) en : | | | | | | |
| - 360 jfa | 1 | 53,6 | 93,5 | 94,2 | 69,1 | 34 |
| - oléfines | 0 | 0 | 0,4 | 0,3 | 3,6 | 6,4 |
| - lourds | 0 | 0,4 | 4,0 | 3,8 | 6,4 | 17,2 |
| T.T. du CCl₄ en CHCl₃ (%) | 0,2 | 0,5 | 0,6 | 1,0 | 2,1 | 1,3 |

| | | | | | | |
|---|---|---|---|---|---|---|
| (*) : 6 heures à 100°C au lieu de 4 heures à 100°C | | | | | | |
| (**) : 2 heures à 100°C au lieu de 4 heures à 100°C | | | | | | |

### EXEMPLE 3

Dans un ballon tricol de 500 ml équipé d'un condenseur à -25°C et d'une ampoule de coulée, on a introduit successivement 1 g (0,01 mole) de chlorure cuivreux, 4,60 g (0,077 mole) d'isopropylamine, puis après agitation, 180,5 g de CCl₄ (1,17 mole). Dans la solution colorée en bleu vif, on a introduit alors 29,6 g (0,38 mole) de 2-chloropropène et on a chauffé le milieu réactionnel à 100°C durant deux heures et trente minutes.

Après arrêt du chauffage et refroidissement durant 6 heures, le mélange réactionnel a été filtré sur "Décalite" jusqu'à disparition de la coloration bleu-verte, puis lavé deux fois à l'acide chlorhydrique 1N pour éliminer les traces d'amine n'ayant pas réagi. La phase organique a ensuite été lavée à l'eau jusqu'à neutralité et séchée sur sulfate de sodium. Le 1,1,1,3,3-pentachlorobutane a ensuite été séparé de l'excès de CCl₄ par distillation sous vide entre la température ambiante et 50°C.

A côté de 1,4 g de 2-chloropropène n'ayant pas réagi (T.T. = 95,3 %), on a obtenu 79 g de 1,1,1,3,3-pentachlorobutane, 1,7 g d'oléfine C₄Cl₄H₄ et 2,7 g de télomères lourds dont 90 % de dimère. La pureté du 360 jfa ainsi obtenu par distillation atteint 98 % et le taux de transformation du 2-chloropropène en 360 jfa est de 88,6 % ; si l'on considère que l'oléfine se fluore en F365 mfc de la même manière que le 360 jfa, le rendement global atteint dans ces conditions 90,8 %.

### EXEMPLE 4

Dans un autoclave en acier inoxydable 316L de 800 ml, équipé d'un indicateur de pression, d'une sonde thermométrique, d'un disque d'éclatement et d'un système d'agitation par barreau magnétique, ont été introduits successivement 41,1 g de 1,1,1,3,3-pentachlorobutane (0,178 mole) et 82,2 g d'acide fluorhydrique anhydre (4,108 moles).

Le réacteur étant chauffé à 100°C, la pression a augmenté graduellement pour atteindre 30,4 bars. Après 8 heures, le système réactionnel a été ramené à température ambiante et une pression résiduelle de 12,8 bars a été observée.

Les composés organiques légers (23,2 g) et les hydracides (96,8 g) ont été éliminés par dégazage puis balayage à l'hélium. Les produits lourds (2 g) ont été collectés après ouverture de l'autoclave.

Les analyses des différentes phases recueillies ont permis de calculer un taux de transformation du 1,1,1,3,3-pentachlorobutane et une sélectivité en 1,1,1,3,3-pentafluorobutane qui atteignent respectivement 98,1 % et 61 %.

Les autres produits de réaction étaient principalement des composés sous-fluorés : C₄H₅ClF₄ (sélectivité = 14,8 %), C₄H₅Cl₂F₃ (2 isomères ; sélectivité = 17,8 %) et C₄H₅Cl₃F₂ (sélectivité = 1 %). Ces composés peuvent être avantageusement recyclés au réacteur, pour être transformés en 1,1,1,3,3-pentafluorobutane.

## Revendications

1. Procédé de préparation du 1,1,1,3,3-pentachlorobutane par addition du tétrachlorure de carbone sur le 2-chloropropène, **caractérisé en ce que** l'on opère en présence d'un sel de cuivre et d'une amine.

2. Procédé selon la revendication 1, dans lequel le sel de cuivre est le chlorure cuivreux.

3. Procédé selon la revendication 1 ou 2, dans lequel l'amine est une amine primaire contenant de 1 à 8 atomes de carbone, de préférence une alkylamine primaire contenant 2 à 4 atomes de carbone.

4. Procédé selon la revendication 3, dans lequel l'amine est l'isopropylamine.

5. Procédé selon l'une des revendications 1 à 4, dans lequel le rapport molaire CCl₄/CH₃CCl = CH₂ est compris entre 2 et 6, de préférence entre 2,5 et 4,5.

6. Procédé selon l'une des revendications 1 à 5, dans lequel le rapport molaire : sel de cuivre/2-chloropropène est compris entre 0,001 et 0,05, de préférence entre 0,005 et 0,02.

7. Procédé selon l'une des revendications 1 à 6, dans lequel la concentration molaire en amine est comprise entre 0,5 et 10 %, de préférence entre 1 et 8 %, et plus particulièrement entre 2,5 et 6 %.

8. Procédé selon l'une des revendications 1 à 7, dans lequel on opère à une température comprise entre 80 et 130°C, de préférence entre 90 et 110°C.

9. Procédé de préparation du 1,1,1,3,3-pentafluorobutane **caractérisé en ce que** l'on soumet un 1,1,1,3,3-pentachlorobutane obtenu par un procédé selon l'une des revendications 1 à 8, à une fluoruration par l'acide fluorhydrique.

10. Procédé selon la revendication 9 dans lequel la fluoruration est effectuée en phase liquide à une température allant d'environ 80 à 120°C, de préférence d'environ 100°C.

11. Procédé selon la revendication 9 ou 10, dans lequel le rapport molaire HF/CCl₃CH₂CCl₂CH₃ est compris entre 15 et 30, de préférence entre 20 et 25.
